# EUROPÄISCHE PATENTSCHRIFT

(11) **EP 2 139 384 B1**
(45) Veröffentlichungstag und Bekanntmachung des Hinweises auf die Patenterteilung: **14.11.2018**
(21) Anmeldenummer: 08748948.0
(22) Anmeldetag: 17.04.2008
(51) Int. Cl.: A61B 5/00, A61M 1/14

(54) **ANORDNUNG AUS MEDIZINISCHEN BEHANDLUNGSEINHEITEN UND PERIPHERIEGERÄTEN SOWIE PERIPHERIEGERÄT UND BEHANDLUNGSEINHEIT ZUR VERWENDUNG IN EINER DERARTIGEN ANORDNUNG**
ASSEMBLY CONSISTING OF MEDICAL TREATMENT UNITS AND PERIPHERAL DEVICES, PERIPHERAL DEVICE AND TREATMENT UNIT FOR USE IN AN ASSEMBLY OF THIS TYPE
ENSEMBLE CONSTITUÉ D'UNITÉS DE TRAITEMENT MÉDICALES ET DE PÉRIPHÉRIQUES AINSI QUE PÉRIPHÉRIQUE ET UNITÉ DE TRAITEMENT À UTILISER DANS UN TEL SYSTÈME

(30) Priorität: 20.04.2007 DE 102007018741
(43) Veröffentlichungstag der Anmeldung: 06.01.2010
(73) Patentinhaber: Fresenius Medical Care Deutschland GmbH, 61352 Bad Homburg v.d.H. (DE)
(72) Erfinder: LEIENDECKER, Claus, 97421 Schweinfurt (DE); MÜLLER, Carsten, 97502 Euerbach (DE); SCHULTE, Elke, 97422 Schweinfurt (DE); ZHANG, Wei, 97464 Niederwerrn (DE)
(74) Vertreter: Oppermann, Frank
(86) Internationale Anmeldenummer: PCT/EP2008/003060
(87) Internationale Veröffentlichungsnummer: WO 2008/128696

(56) Entgegenhaltungen:
- EP-A- 1 574 164
- US-A- 5 827 180
- US-A1- 2006 025 663

## Beschreibung

Die Erfindung betrifft eine Anordnung aus mindestens zwei Blutbehandlungsvorrichtungen und mindestens zwei Peripheriegeräten, wobei eine medizinische Blutbehandlungsvorrichtung der mindestens zwei Blutbehandlungsvorrichtungen und ein Peripheriegerät der mindestens zwei Peripheriegeräte einem Patienten und das dem Patienten zuzuordnende Peripheriegerät der dem Patienten zuzuordnenden Blutbehandlungsvorrichtung zuzuordnen ist.

In der Medizintechnik finden zur Behandlung vom Patienten unterschiedliche Behandlungseinheiten Verwendung, an den die Patienten bzw. die an den Patienten angeschlossen werden. Die Verbindung zwischen Patient und Maschine erfolgt im Allgemeinen über Schläuche und/oder Leitungen. Zu den bekannten Behandlungseinheiten zählen beispielsweise Dialysemaschinen mit einem extrakorporalen Blutkreislauf.

Es ist bekannt, medizinische Behandlungseinheiten zusammen mit einem oder mehreren Peripheriegeräten zu betreiben. Derartige Satelliten dienen beispielsweise dazu, während der Behandlung den Patienten zu überwachen, wobei die ermittelten patientenspezifischen Daten an die Behandlungseinheit übertragen werden.

Während der Dialyse beispielsweise werden physiologische Daten des Dialysepatienten mit verschiedenen Sensoren erfasst. Dabei wird angestrebt, dass die Verbindung zwischen Patient und Dialysemaschine sowie dem Peripheriegerät nur aus den erforderlichen Blutschläuchen des extrakorporalen Blutkreislaufs und möglichst wenigen weiteren Leitungen besteht.

Während zwischen der Behandlungseinheit und dem Patienten über die Schläuche eine feste Verbindung besteht, kann die Datenübertragung zwischen dem Peripheriegerät und der Behandlungseinheit drahtlos erfolgen, beispielsweise per Funk- oder Lichtsignale. Folglich kann das Bedienpersonal anhand der festen Verbindung zwar sofort die Zuordnung zwischen Behandlungseinheit und Patient erkennen, nicht aber die Zuordnung zwischen Peripheriegerät und Behandlungseinheit.

Wenn eine Mehrzahl von Behandlungseinheiten und Peripheriegeräten zusammen in einem Behandlungsraum betrieben werden, ist es erforderlich, jeweils eine Behandlungseinheit und ein Peripheriegerät einem Patienten zuzuordnen und eine Verbindung von dem jeweiligen Peripheriegerät zu der jeweiligen Behandlungseinheit herzustellen.

Eine falsche Zuordnung zwischen dem Peripheriegerät und der Behandlungseinheit einerseits und dem Peripheriegerät und dem Patienten andererseits kann im Extremfall zu lebensbedrohlichen Komplikationen während der Behandlung führen. Dies ist insbesondere dann besonders problematisch, wenn aufgrund einer kabellosen Verbindung die falsche Zuordnung nicht sofort erkannt werden kann.

Wenn mehrere Geräte gleichzeitig betrieben werden, die miteinander kommunizieren, finden im Allgemeinen so genannte Identifikations-(ID)-Signale Verwendung, mit denen erkannt werden kann, ob die Signale des einen oder anderen Satelliten empfangen werden. Die US 6,332,094 B1 beispielsweise beschreibt einen Pulsmesser, der die Pulssignale zusammen mit einem Identifikationssignal an einen Empfänger drahtlos überträgt.

Die WO 2004/056263 A1 beschreibt ein Verfahren zur drahtlosen Übertragung von Signalen zwischen mehreren Peripheriegeräten und mehreren Behandlungseinheiten. Den Behandlungseinheiten sind mehrere Empfänger zugeordnet, die die Signale der Peripheriegeräte empfangen. Die Empfangsbereitschaft des Empfängers für Signale der Peripheriegeräte wird dadurch hergestellt, dass sich das Peripheriegerät beim Empfänger anmeldet. Nur im Zustand der Empfangsbereitschaft setzt der Empfänger die Signale des Peripheriegeräts um, die der jeweiligen Behandlungseinheit übermittelt werden.

Aus der US 6,870,475 B2 ist eine Ladestation zum Aufladen des Akkus einer portablen medizinischen Überwachungseinheit bekannt, die der Überwachung von patientenspezifischen Daten dient. Das Patientenüberwachungssystem sieht eine Vielzahl von Ladestationen an unterschiedlichen Orten vor, in die der Patient die Überwachungseinheit zum Laden des Akkus einsetzen kann. Patientenspezifische Daten der portablen Überwachungseinheiten können drahtlos an eine zentrale Überwachungseinheit übertragen werden.

Die US 6,184,651 B1 und US 5,455,466 beschreiben allgemein Ladestationen zum Aufladen des Akkus von elektrischen Geräten, wobei eine induktive Kopplung zwischen dem elektrischen Gerät und der Ladestation erfolgt.

Die US 2006/025663 A1 beschreibt ein Überwachungssystem zur Überwachung des Blutzuckerspiegels eines Patienten. Das Überwachungssystem verfügt über einen Blutzucker-Sensor, eine Benutzer-Schnittstelle und einen Patientenmonitor. Die Benutzer-Schnittstelle weist Eingabe- und Ausgabeeinheiten auf, die eine Kommunikation mit dem Blutzucker-Sensor erlauben. Der Patientenmonitor, der mit der Benutzer-Schnittstelle kommunizieren kann, dient der Überwachung des Patienten auf der Behandlungsstation in der Klinik. In der US 2006/025663 A1 wird eine Anordnung von mehreren Überwachungssystemen beschrieben, die sich auf der Station befinden. Die Kommunikation zwischen den Geräten kann drahtlos erfolgen. Den Sensoren, Benutzer-Schnittstellen und Patientenmonitoren sind jeweils Identifikationscodes oder Benutzernamen (ID) zugeordnet, so dass einen Verbindung nur zwischen den zugehörigen Geräten aufgebaut werden kann.

Der Erfindung liegt die Aufgabe zugrunde, die Sicherheit und Flexibilität beim Betrieb von mindestens zwei Blutbehandlungsvorrichtungen und Peripheriegeräten zu erhöhen.

Die Lösung dieser Aufgabe erfolgt erfindungsgemäß mit den Merkmalen des Patentanspruchs 1. Bevorzugte Ausführungsformen sind Gegenstand der Unteransprüche.

Die Sicherheit und Flexibilität beim Betrieb von einer Mehrzahl von medizinischen Blutbehandlungsvorrichtungen und Peripheriegeräten wird bei der erfindungsgemäßen Anordnung dadurch erhöht, dass das Bedienpersonal nach der Zuordnung einer Blutbehandlungsvorrichtung zu einem Patienten und eines Peripheriegeräts zu einem Patienten bestätigt, dass der Patient, dem das Peripheriegerät zugeordnet worden ist, derjenige Patient ist, dem diejenige Blutbehandlungsvorrichtung zugeordnet worden ist, der das Peripheriegerät zugeordnet worden ist. Das Peripheriegerät wird nur unter der Bedingung freigegeben, dass die richtige Zuordnung von Peripheriegerät und Patient bestätigt worden ist. Dadurch wird ausgeschlossen, dass zwar das richtige Peripheriegerät der richtigen Blutbehandlungsvorrichtung zugeordnet ist, aber das der Blutbehandlungsvorrichtung zugeordnete Peripheriegerät dem falschen Patienten zugeordnet ist.

Die Mittel zur Freigabe des Peripheriegeräts können derart ausgebildet sein, dass die patientenspezifischen Daten erst dann an die Blutbehandlungsvorrichtung übertragen oder von der Blutbehandlungsvorrichtung empfangen oder ausgewertet werden, wenn die richtige Zuordnung von Peripheriegerät und Patient bestätigt worden ist. Die Mittel zur Freigabe des Peripheriegeräts können beispielsweise auch derart ausgebildet sein, dass die medizinische Behandlung solange nicht in Gang gesetzt werden kann, wie nicht die richtige Zuordnung von Peripheriegerät und Patient bestätigt worden ist.

Bei einer bevorzugten Ausführungsform der Erfindung weisen die Mittel zum Zuordnen eines Peripheriegeräts zu einer Blutbehandlungsvorrichtung Mittel zum Senden einer Adresse zur Identifikation des Peripheriegeräts an die Blutbehandlungsvorrichtung und Mittel zum Empfangen der Adresse zur Identifikation des Peripheriegeräts von der Blutbehandlungsvorrichtung auf. Beispielsweise kann die Adresse eine MAC-Adresse (Media Access Control) sein, die zur eindeutigen Identifikation des Peripheriegeräts im Netzwerk dient.

Eine weitere bevorzugte Ausführungsform sieht vor, dass die Mittel zum Zuordnen von Peripheriegerät und Blutbehandlungsvorrichtung Mittel zur Aufnahme eines Peripheriegeräts und Mittel zum Erkennen eines in den Mitteln zur Aufnahme eines Peripheriegeräts aufgenommenen Peripheriegeräts aufweisen. Die Mittel zur Aufnahme eines Peripheriegeräts können eindeutig einer Blutbehandlungsvorrichtung zugeordnet werden, wobei diese Zuordnung sofort vom Bedienpersonal erkannt werden kann. Beispielsweise sind die Mittel zur Aufnahme eines Peripheriegeräts in enger räumlicher Nähe zur Blutbehandlungsvorrichtung angeordnet oder Bestandteil der Blutbehandlungsvorrichtung. Das Peripheriegerät ist dann der Blutbehandlungsvorrichtung eindeutig zugeordnet, wenn sich das Peripheriegerät in den der Blutbehandlungsvorrichtung zugeordneten Mitteln zur Aufnahme eines Peripheriegeräts befindet.

Die Mittel zum Aufnahme eines Peripheriegeräts können unterschiedlich ausgebildet sein. Beispielsweise können die Mittel zur Aufnahme eines Peripheriegeräts eine Aufnahmeeinheit sein, in die das Peripheriegerät eingelegt wird. Das Peripheriegerät kann in die Aufnahmeeinheit lose eingelegt oder auch in der Aufnahmeeinheit gesichert sein. Auch die Mittel zum Erkennen des Peripheriegeräts in der Aufnahmeeinheit können unterschiedlich ausgebildet sein. Beispielsweise können die Mittel zum Erkennen des Peripheriegeräts mechanische Kontaktgeber aufweisen, mit denen erkannt werden kann, ob sich das Peripheriegerät in der Aufnahmeeinheit befindet. Alternativ können die Mittel zum Erkennen des Peripheriegeräts über optische oder induktive Sensoren verfügen.

Eine besonders bevorzugte Ausführungsform, die in der Praxis große Vorteile bietet, sieht vor, dass die Mittel zur Aufnahme eines Peripheriegeräts Mittel zum Aufladen eines Akkus zur Stromversorgung des Peripheriegeräts aufweisen. Die Aufnahmeeinheiten können als Ladestationen für die Peripheriegeräte ausgebildet sein. Dadurch stellen die Aufnahmeeinheiten nicht nur die Zuordnung von Peripheriegerät und Blutbehandlungsvorrichtung, sondern auch den dauerhaften Betrieb der Peripheriegeräte sicher.

Bei einer weiteren besonders bevorzugten Ausführungsform sendet das Peripheriegerät die Adresse zur Identifikation an die Blutbehandlungsvorrichtung dann, wenn das Peripheriegerät in der Aufnahmeeinheit erkannt worden ist. Mit dem Einlegen des Peripheriegeräts in die Aufnahmeeinheit erfolgt somit vollautomatisch die Identifikation des Peripheriegeräts. Es ist aber auch möglich, dass das Peripheriegerät die Identifikationsadresse dauerhaft sendet und die Blutbehandlungsvorrichtung die Identifikationsadresse erst nach dem Erkennen des Peripheriegeräts in der Aufnahmeeinheit auswertet.

Die erfolgreiche Zuordnung von Peripheriegerät und Patient signalisiert das Peripheriegerät der Blutbehandlungsvorrichtung dadurch, dass das Peripheriegerät ein entsprechendes Signal an die Blutbehandlungsvorrichtung sendet. Hierfür verfügen die Mittel zum Überprüfen der Zuordnung von Peripheriegerät und Patient über Mittel zum Senden und Empfangen von Signalen, insbesondere Mittel zur drahtlosen Übertragung von Signalen, beispielsweise einen Funk-Sender und einen Funk-Empfänger. Es sind aber auch andere drahtlose Übertragungsstrecken, beispielsweise eine optische Datenübertragung möglich.

Auf eine erfolgreiche Zuordnung von Peripheriegerät und Patient wird vorzugsweise dann geschlossen, wenn das Peripheriegerät patientenspezifische Daten zur Überwachung des Patienten empfängt, was voraussetzt, dass das Peripheriegerät an den Patient angeschlossen ist. Es ist aber auch möglich, zusätzliche Sensoren vorzusehen, die die Lage des Peripheriegeräts erkennen, beispielsweise Kontaktgeber an den Schläuchen und/oder Leitungen.

Die Sicherheit kann weiterhin dadurch erhöht werden, dass das Bedienpersonal aufgefordert wird, innerhalb eines vorgegebenen Zeitintervalls das der Aufnahmeeinheit entnommene Peripheriegerät, das der Blutbehandlungsvorrichtung zugeordnet ist, dem Patienten zuzuordnen. Eine besonders bevorzugte Ausführungsform sieht daher vor, dass die Mittel zum Überprüfen der Zuordnung von Peripheriegerät und Patient mit Mitteln zum Messen der Zeit nach der Entnahme des Peripheriegeräts aus dem Mitteln zur Aufnahme des Peripheriegeräts zusammenwirken. Vorzugsweise wirken die Mittel zum Überprüfen der Zuordnung von Peripheriegerät und Patient mit Mitteln zur Anzeige der verbleibenden Zeit in dem vorgegeben Zeitintervall zusammen, um dem Bedienpersonal zu signalisieren, wie viel Zeit noch bleibt, um das Peripheriegerät an den Patienten anzuschließen.

Wenn das Peripheriegerät erst nach Ablauf des vorgegebenen Zeitintervalls dem Patienten zugeordnet werden sollte, wird die erfolgreiche Zuordnung von Peripheriegerät und Patient nicht der Blutbehandlungsvorrichtung signalisiert. Das Bedienpersonal ist damit gezwungen, das Peripheriegerät wieder in die Aufnahmeeinheit einzulegen, um erneut die Zuordnung von Peripheriegerät, Blutbehandlungsvorrichtung und Patient in dem vorgegebenen Zeitintervall vornehmen zu können. Dadurch wird verhindert, dass das der Aufnahmeeinheit entnommene Peripheriegerät vom Bedienpersonal für unbestimmte Zeit herumgetragen wird, da in diesem Fall ein besonders großes Risiko einer falschen Zuordnung von Peripheriegerät und Patient besteht.

Die Aufforderung nach Ablauf des vorgegebenen Zeitintervalls das Peripheriegerät wieder in die Aufnahmeeinheit einzulegen, erfolgt vorzugsweise mit Mitteln zum Geben eines optischen und/oder akustischen Signals, das die Mittel zum Überprüfen der Zuordnung für Peripheriegerät und Patient vorzugsweise aufweisen.

Die Mittel zum Bestätigen der Zuordnung von Blutbehandlungsvorrichtung, Peripheriegerät und Patient weisen vorzugsweise Mittel zur manuellen Eingabe auf, beispielsweise Schalter, Taster etc. Die Mittel zur manuellen Eingabe können auch als Sensoren ausgebildet sein, die berührungslos arbeiten, beispielsweise Induktionsschalter etc.

Die Erfindung geht davon aus, dass die Zuordnung von Blutbehandlungsvorrichtung und Patient eine feste Verbindung von Patient und Blutbehandlungsvorrichtung voraussetzt, so dass diese Zuordnung für das Bedienpersonal eindeutig ist. Da es sich bei der Behandlungseinheit um eine Blutbehandlungsvorrichtung handelt, ist die feste Zuordnung von Blutbehandlungsvorrichtung und Patient durch das Blutschlauchsystem gegeben, so dass Verwechselungen in der Praxis ausgeschlossen sind.

Die Peripheriegeräte können unterschiedlich ausgebildet sein und unterschiedliche Funktionen haben. Beispielsweise können die Peripheriegeräte eine oder mehrere Sensoren zum Erfassen von patientenspezifischen Daten, beispielsweise zum Messen von physiologischen Messwerten, wie Blutdruck oder Puls etc. aufweisen.

Im Folgenden wird ein Ausführungsbeispiel der Erfindung unter Bezugnahme auf die Zeichnungen im Einzelnen erläutert.

Es zeigen:
- Fig. 1: Eine Prinzipdarstellung einer korrekten Zuordnung zwischen zwei Peripheriegeräten und Blutbehandlungsvorrichtungen zu dem jeweiligen Patienten,
- Fig. 2: die Anordnung von Fig. 1, wobei die der jeweiligen Blutbehandlungsvorrichtung zugeordneten Peripheriegeräte dem falschen Patienten zugeordnet worden sind,
- Fig. 3: die wesentlichen Komponenten einer Blutbehandlungsvorrichtung und eines Peripheriegeräts in schematischer Darstellung und
- Fig. 4A und 4B: ein Verlaufsdiagramm, das den Ablauf der Zuordnung von Peripheriegerät und Blutbehandlungsvorrichtungen sowie Patient beschreibt.

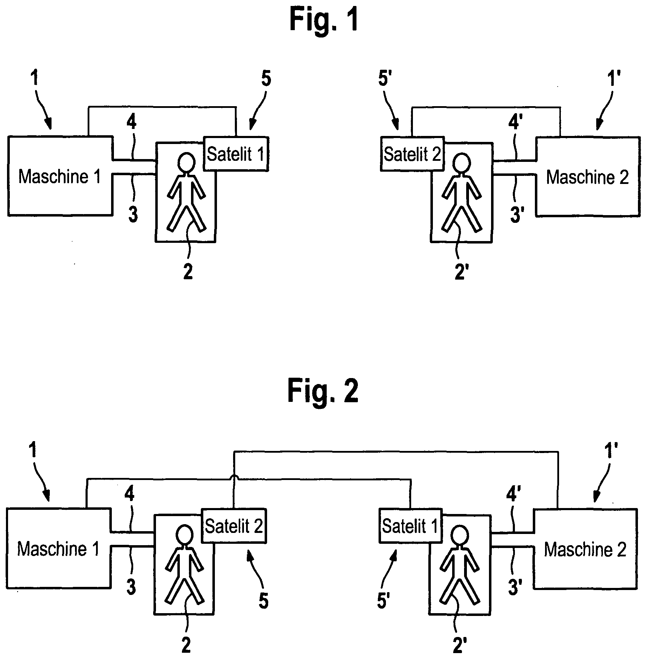

Fig. 1 zeigt zwei medizinische Behandlungseinheiten 1, 1', die extrakorporale Blutbehandlungsvorrichtungen mit einem extrakorporalen Blutkreislauf sind. An jeder Blutbehandlungsvorrichtung 1, 1' ist ein Patient 2, 2' angeschlossen. Für den Fall einer extrakorporalen Blutbehandlung ist der Patient 2, 2' mit der Blutbehandlungsvorrichtung 1, 1' über eine venöse und arterielle Schlauchleitung 3, 4; 3', 4' verbunden. Damit ist eine feste Zuordnung zwischen dem Patienten und der Blutbehandlungsvorrichtung getroffen. Darüber hinaus ist den beiden Patienten 2, 2' jeweils ein Peripheriegerät 5, 5', beispielsweise ein Blutdruckmonitor zugeordnet.

Die Peripheriegeräte 5, 5' überwachen die Körperfunktion des Patienten, beispielsweise den Blutdruck oder Puls, und übertragen die physiologischen Daten an die zugehörige Blutbehandlungsvorrichtung, an die der Patient angeschlossen ist.

Fig. 1 zeigt die richtige Zuordnung von Peripheriegerät und Patient einerseits und Peripheriegerät und Blutbehandlungsvorrichtung andererseits, während Fig. 2 eine falsche Zuordnung von Peripheriegerät und Patient bzw. Peripheriegerät und Blutbehandlungsvorrichtung zeigt. Bei einer falschen Zuordnung empfängt die Blutbehandlungsvorrichtung 1 die Daten des Peripheriegeräts 5' des Patienten 2', der nicht an die Blutbehandlungsvorrichtung 1, sondern an die Blutbehandlungsvorrichtung 1' angeschlossen ist.

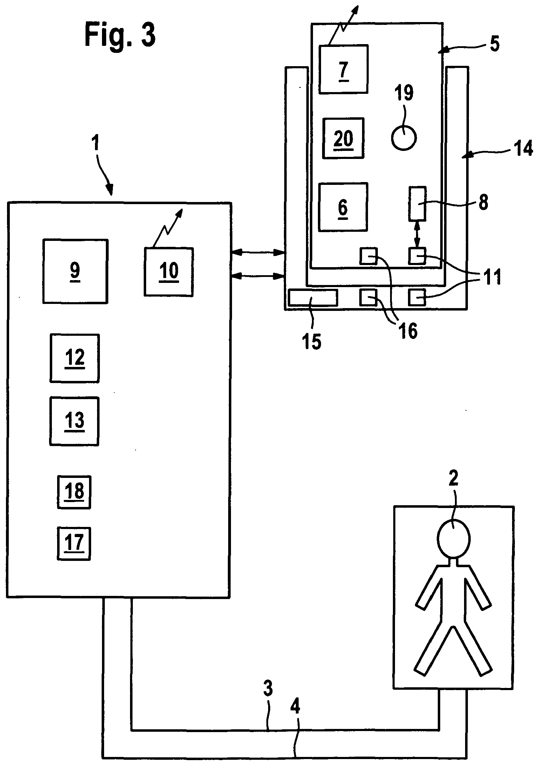

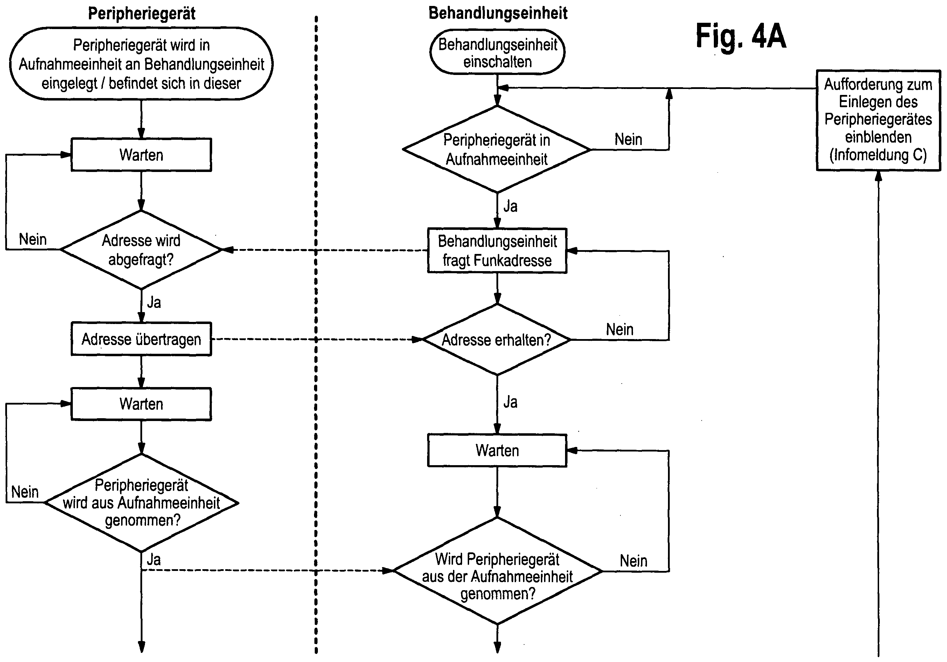

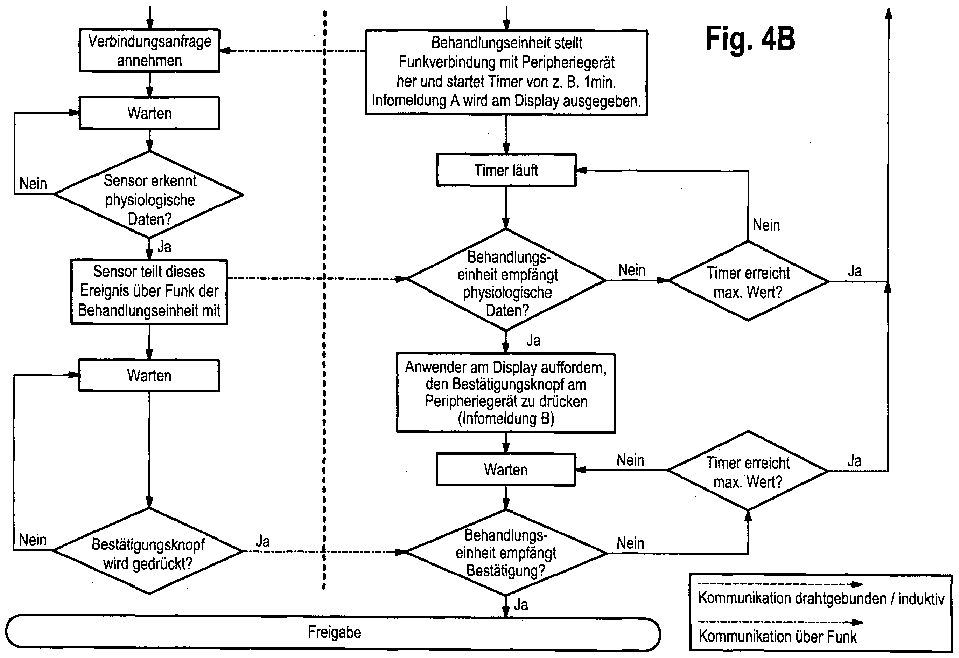

Die erfindungsgemäße Anordnung umfasst mindestens zwei Blutbehandlungsvorrichtungen und Peripheriegeräte. Im Folgenden werden die einzelnen Komponenten einer Blutbehandlungsvorrichtung und eines Peripheriegeräts sowie die einzelnen Schritte für die Zuordnung von Blutbehandlungsvorrichtung, Peripheriegerät und Patient unter Bezugnahme auf die Figuren 3 und 4A, 4B beschrieben.

Das Peripheriegerät 5, beispielsweise ein Blutdruckmonitor, verfügt über verschiedene nur schematisch dargestellte Sensoren 6 zur Überwachung des Patienten, beispielsweise Sensoren zum Messen des Blutdrucks oder Pulses. Zur bidirektionalen Kommunikation mit der zugehörigen Blutbehandlungsvorrichtung 1 verfügt das Peripheriegerät 5 über Mittel 7 zum Senden und Empfangen von Signalen, insbesondere einen Funk-Sender/Empfänger 7. Die Datenübertragung kann aber auch nur direktional von dem Peripheriegerät zu der Blutbehandlungsvorrichtung erfolgen. Der Stromversorgung des Peripheriegeräts dient eine wieder aufladbare Batterie 8 (Akku).

Des Weiteren weist das Peripheriegerät 5 Mittel 19 auf, mit dem das Bedienpersonal bestätigen kann, dass der Patient, dem das Peripheriegerät zugeordnet worden ist, derjenige Patient ist, dem diejenige Blutbehandlungsvorrichtung zugeordnet worden ist, der das Peripheriegerät zugeordnet worden ist. Bei diesen Mitteln handelt es sich vorzugsweise um Mittel zur manuellen Eingabe, insbesondere einen Taster 19.

Die Blutbehandlungsvorrichtung 1 mit einem extrakorporalen Blutkreislauf verfügt über verschiedene nur schematisch dargestellte Komponenten 9 zur Behandlung des Patienten 2, beispielsweise einen Dialysator, Pumpen etc. Der Patient 2 ist mit der Blutbehandlungsvorrichtung 1 fest verbunden, beispielsweise über eine venöse und arterielle Blutleitung 3, 4. Damit ist eine feste Zuordnung zwischen Patient und Blutbehandlungsvorrichtung gegeben.

Zur Kommunikation mit dem zugehörigen Peripheriegerät 5 verfügt die Blutbehandlungsvorrichtung 1 über Mittel 10 zum Senden und Empfangen von Signalen, insbesondere einen Funk-Sender/Empfänger 10 für eine bidirektionale Datenübertragung. Die Datenübertragung zwischen Peripheriegerät 5 und Blutbehandlungsvorrichtung 1 erfolgt also per Funk, wobei die hierzu erforderlichen Sender und Empfänger sowie Schnittstellen dem Fachmann allgemein bekannt sind.

Darüber hinaus verfügt die Blutbehandlungsvorrichtung 1 über einen optischen und/oder akustischen Alarmgeber 12 und eine Anzeigeeinheit 13.

Jeder Blutbehandlungsvorrichtung 1 ist eine Aufnahmeeinheit 14 fest zugeordnet, so dass für das Bedienpersonal sofort erkennbar ist, dass die Aufnahmeeinheit 14 zu der Blutbehandlungsvorrichtung 1 gehört. Die Aufnahmeeinheit 14 ist als Ladestation ausgebildet. Sie weist Mittel 15 zur Stromversorgung, beispielsweise ein Netzteil auf, mit dem der Akku 8 des Peripheriegeräts geladen werden kann, wenn das Peripheriegerät in die Aufnahmeeinheit eingelegt wird. Die elektrische Verbindung zwischen dem Netzteil 15 und dem Akku 8 kann über geeignete Steckverbindungen 11 oder eine induktive Kopplung erfolgen.

Das Peripheriegerät 5 und/oder die Aufnahmeeinheit 14 verfügen über Mittel 16 zum Erkennen, ob ein Peripheriegerät in die Aufnahmeeinheit eingelegt ist. Diese Mittel können beispielsweise elektrische Kontaktgeber sein, die entweder an dem Peripheriegerät oder der Aufnahmeeinheit oder an beiden Geräten vorgesehen sind.

Nachfolgend wird die Funktion der Blutbehandlungsvorrichtungen und Peripheriegeräte unter Bezugnahme auf die Figuren 3 sowie 4A, 4B im Einzelnen beschrieben.

Es wird davon ausgegangen, dass die Patienten an die Blutbehandlungsvorrichtungen 1 über die venösen und arteriellen Blutleitungen 3, 4 angeschlossen sind, so dass jeweils eine feste Zuordnung von Patient und Behandlungseinheit gegeben ist, die für das Bedienpersonal sofort ersichtlich ist. Das Bedienpersonal hat nun die Aufgabe, das zu der Behandlungseinheit gehörende Peripheriegerät an den jeweiligen Patienten anzuschließen.

Zunächst wird das jeweilige Peripheriegerät 5 in die Aufnahmeeinheit 14 eingelegt, die der jeweiligen Blutbehandlungsvorrichtung 1 zugeordnet ist. Damit hat das Bedienpersonal das Peripheriegerät der Blutbehandlungsvorrichtung zugeordnet.

Die Mittel 16 erkennen, dass sich das Peripheriegerät in der Aufnahmeeinheit 14 befindet. Dies signalisieren die Mittel 16 mit einem Statussignal. Während sich das Peripheriegerät 5 in der Aufnahmeeinheit 14 befindet, ist eine elektrische Verbindung zwischen dem Netzteil 15 und dem Akku 8 hergestellt, so dass der Akku geladen wird.

Die Blutbehandlungsvorrichtung 1 fragt fortlaufend an, ob das Statussignal, dass sich das Peripheriegerät in der Aufnahmeeinheit befindet, erzeugt worden ist. Wenn dies der Fall ist, sendet der Funk-Sender/Empfänger 7 des Peripheriegeräts 5 eine Adresse zur Identifikation des Peripheriegeräts, beispielsweise eine MAC-Adresse an die Blutbehandlungsvorrichtung 1, die die MAC-Adresse mit dem Funk-Sender/Empfänger 10 empfängt. Damit hat die Blutbehandlungsvorrichtung 1 das Peripheriegerät 5 identifiziert.

Für die Identifizierung der Peripheriegeräte durch Austausch der MAC-Adressen finden im Falle einer drahtlosen Übermittlung der Signale vorzugsweise Sende-/Empfangsmittel mit einer relativ kurzen Reichweite Verwendung. Wenn die Reichweite der Sende-/Empfangsmittel nicht größer als der Abstand zwischen Peripheriegerät und der Blutbehandlungsvorrichtung mit der zugehörigen Aufnahmeeinheit ist, kann sichergestellt werden, dass das Peripheriegerät nur mit der "eigenen Ladeschale" kommuniziert. Hierzu können die Sendemittel auch selbst die Reichweite begrenzen. Es können aber auch getrennte Sende-Empfangsmittel für die Übertragung der patientenspezifischen Signale einerseits und der MAC-Adressen zur Identifikation der Peripheriegeräte andererseits vorgesehen sein. Beispielsweise sind für die Identifikation auch Sende-/Empfangsmittel zur induktiven Datenübertragung möglich, die Teil eines in der Ladeschale vorgesehenen induktiven Akkuladegeräts sind.

Nach der Identifikation des Peripheriegeräts muss das Bedienpersonal das identifizierte Peripheriegerät 5 dem Patienten zuordnen, der an die Blutbehandlungsvorrichtung 1, die das Peripheriegerät identifiziert hat, angeschlossen ist. Hierzu muss das Bedienpersonal das Peripheriegerät aus der Aufnahmeeinheit nehmen.

Es wird fortlaufend überwacht, ob sich das Peripheriegerät noch in der Aufnahmeeinheit befindet. Wenn das Bedienpersonal das Peripheriegerät aus der Aufnahmeeinheit nimmt, wird dies von den Mitteln 16 sofort erkannt, die ein Statussignal erzeugen, dass das Bedienpersonal das Peripheriegerät der Aufnahmeeinheit entnommen hat. Dieses Statussignal wird an die Blutbehandlungsvorrichtung gesendet, die das Statussignal empfängt.

Mit dem Empfang des Statussignals für das Entnehmen des Peripheriegeräts aus der Aufnahmeeinheit setzt die Blutbehandlungsvorrichtung Mittel (Timer) 18 zum Messen der Zeit in Gang, die nach der Entnahme des Peripheriegeräts verstrichen ist.

Das Bedienpersonal muss nun innerhalb eines vorgegebenen Zeitintervalls das Peripheriegerät an den Patienten anschließen. Die verbleibende Zeit in dem vorgegebenen Zeitintervall wird auf einer Anzeigeeinheit 13 der Blutbehandlungsvorrichtung 1 dem Bedienpersonal angezeigt.

Wenn das Bedienpersonal das Peripheriegerät an dem Patienten angebracht hat und das Peripheriegerät aktiviert hat, empfangen die Sensoren 6 des Peripheriegeräts die physiologischen Messwerte, wie Blutdruck und Puls des Patienten. Die von den Sensoren 6 empfangenen Signale werten Mittel 20 zum Überprüfen der Zuordnung eines Peripheriegeräts zu einem Patienten aus. Dieses Ereignis signalisieren die Mittel 20 des Peripheriegeräts 5 der Blutbehandlungsvorrichtung 1 dadurch, dass das Peripheriegerät an die Blutbehandlungsvorrichtung ein Statussignal für die erfolgreiche Zuordnung des Peripheriegeräts zu dem Patienten sendet.

Die Blutbehandlungsvorrichtung prüft fortlaufend, ob das Statussignal für die erfolgreiche Zuordnung von Peripheriegerät und Patient während des vorgegebenen Zeitintervalls empfangen wird. Wenn das vorgegebene Zeitintervall vor dem Empfang des Statussignals für die erfolgreiche Zuordnung von Peripheriegerät und Patient abgelaufen ist, gibt die Blutbehandlungsvorrichtung mit dem Alarmgeber 12 ein optisches und/oder akustisches Alarmsignal, mit dem das Bedienpersonal aufgefordert wird, das Peripheriegerät wieder in die Aufnahmeeinheit einzulegen. Gleichzeitig oder alternativ kann auch auf der Anzeigeeinheit 13 eine entsprechende Anzeige erfolgen. Dann beginnt die oben beschriebene Routine von neuem.

Wird jedoch das Statussignal für die erfolgreiche Zuordnung von Peripheriegerät und Patient empfangen, erscheint auf der Anzeigeeinheit 13 (Display) der Blutbehandlungsvorrichtung ein Hinweis, der das Bedienpersonal auffordert, den Bestätigungsknopf 19 zu drücken. Die Blutbehandlungsvorrichtung wartet ab, bis der Knopf 19 gedrückt wird.

Das Bedienpersonal hat nun zu überprüfen, ob der Patient 2, an den das Peripheriegerät 5 angeschlossen worden ist, derjenige Patient ist, an dem die Blutbehandlungsvorrichtung 1 angeschlossen ist, der das Peripheriegerät durch die Aufnahmeeinheit 14 zugeordnet worden ist. Wenn dies der Fall ist, drückt das Bedienpersonal den Bestätigungsknopf 19, der an dem Peripheriegerät vorgesehen ist. Dann wird ein Statussignal für die Bestätigung durch das Bedienpersonal an die Blutbehandlungsvorrichtung gesendet, die das Statussignal empfängt.

Es ist aber auch möglich, dass sich der Bestätigungsknopf an der Blutbehandlungsvorrichtung befindet. Dann ist es nicht erforderlich, das Statussignal für die Bestätigung der Zuordnung von Peripheriegerät und Patient an die Blutbehandlungsvorrichtung zu senden.

Die Blutbehandlungsvorrichtung 1 überprüft nunmehr, ob innerhalb des vorgegebenen Zeitintervalls das Bedienpersonal die korrekte Zuordnung von Peripheriegerät und Patient durch Drücken des Knopfes 19 bestätigt hat.

Die Blutbehandlungsvorrichtung 1 weist Mittel 17 auf, mit denen das Peripheriegerät freigegeben wird. Nur für den Fall, dass der Bestätigungsknopf 19 innerhalb des vorgegebenen Zeitintervalls gedrückt worden ist, geben die Mittel 17 das Peripheriegerät frei, so dass die patientenspezifischen Daten von dem Peripheriegerät an die Blutbehandlungsvorrichtung mit den Funk-Sender/Empfängern 7, 10 übertragen werden. Damit geben die Mittel 17 auch die Behandlung frei. Das Behandlungsprogramm kann nunmehr gestartet werden.

Die oben beschriebenen Mittel können separate Baugruppen bilden, in der Praxis sind die Mittel aber Bestandteil eines Mikroprozessors, der in den Blutbehandlungsvorrichtungen und Peripheriegeräten im Allgemeinen ohnehin vorgesehen ist.

Bei dem unter Bezugnahme auf die Figuren beschriebenen Ausführungsbeispiel sind einzelne Mittel jeweils den Blutbehandlungsvorrichtungen und einzelne Mittel jeweils den Peripheriegeräten zugeordnet. Die Mittel, die den Blutbehandlungsvorrichtungen zugeordnet sind, können aber auch den Peripheriegeräten zugeordnet werden. Beispielsweise können die Mittel zur Bestätigung der Zuordnung von Patient und Blutbehandlungsvorrichtung nicht an dem Peripheriegerät, sondern an der Blutbehandlungsvorrichtung vorgesehen sein. Auch können die Mittel zum Geben eines akustischen und/oder optischen Alarms anstelle an der Blutbehandlungsvorrichtung an dem Peripheriegerät vorgesehen sein.

Auch wenn die Figuren eine Anordnung aus Peripheriegeräten und Blutbehandlungsvorrichtungen zeigen, ist aus der Beschreibung ersichtlich, dass die Peripheriegeräte und die Blutbehandlungsvorrichtungen selbständige Geräte sind, die über die beschriebenen Baugruppen verfügen.

## Patentansprüche

1. Anordnung aus mindestens zwei Blutbehandlungsvorrichtungen mit einem extrakorporalen Blutkreislauf und mindestens zwei Peripheriegeräten, die jeweils mindestens einen Sensor (6) zum Messen von physiologischen Daten des Patienten aufweisen, wobei eine Blutbehandlungsvorrichtung (1) der mindestens zwei Blutbehandlungsvorrichtungen und ein Peripheriegerät (5) der mindestens zwei Peripheriegeräte einem Patienten und das dem Patienten zuzuordnende Peripheriegerät der dem Patienten zuzuordnenden Blutbehandlungsvorrichtung zuzuordnen ist,
wobei die Peripheriegeräte und die Blutbehandlungsvorrichtungen aufweisen:
Mittel (3, 4) zum Zuordnen einer Blutbehandlungsvorrichtung zu einem Patienten, die ein System von Schläuchen und/oder Leitungen zum Anschließen des Patienten an die Blutbehandlungsvorrichtung aufweisen,
Mittel (7, 10, 14, 16) zum Zuordnen eines Peripheriegeräts zu einer Blutbehandlungsvorrichtung,
Mittel (20) zum Überprüfen der Zuordnung eines Peripheriegeräts zu einem Patienten, die Mittel (7) zum Senden eines Signals an die Blutbehandlungsvorrichtung nach der erfolgreichen Zuordnung des Peripheriegeräts zu dem Patienten und Mittel (10) zum Empfangen des Signals von der Blutbehandlungsvorrichtung aufweisen,
Mittel (19) zum Bestätigen, dass der Patient, dem das Peripheriegerät zugeordnet worden ist, derjenige Patient ist, dem diejenige Blutbehandlungsvorrichtung zugeordnet worden ist, der das Peripheriegerät zugeordnet worden ist und
Mittel (17) zur Freigabe des Peripheriegeräts nur unter der Bedingung, dass die richtige Zuordnung von Peripheriegerät und Patient bestätigt worden ist.

2. Anordnung nach Anspruch 1, **dadurch gekennzeichnet, dass** die Mittel zum Zuordnen eines Peripheriegeräts zu einer Blutbehandlungsvorrichtung Mittel (7) zum Senden einer Adresse zur Identifikation des Peripheriegeräts an die Blutbehandlungsvorrichtung und Mittel (10) zum Empfangen der Adresse zur Identifikation des Peripheriegeräts von der Blutbehandlungsvorrichtung aufweisen.

3. Anordnung nach Anspruch 1 oder 2, **dadurch gekennzeichnet, dass** die Mittel zum Zuordnen eines Peripheriegeräts zu einer Blutbehandlungsvorrichtung Mittel (14) zur Aufnahme eines Peripheriegeräts und Mittel (16) zum Erkennen eines in den Mitteln zur Aufnahme eines Peripheriegeräts aufgenommenen Peripheriegeräts aufweisen.

4. Anordnung nach Anspruch 3, **dadurch gekennzeichnet, dass** die Mittel (14) zur Aufnahme eines Peripheriegeräts eine Aufnahmeeinheit sind, in die das Peripheriegerät eingelegt wird.

5. Anordnung nach Anspruch 3 oder 4, **dadurch gekennzeichnet, dass** die Mittel (14) zur Aufnahme eines Peripheriegeräts Mittel (15) zum Aufladen eines Akkus (8) zur Stromversorgung des Peripheriegeräts aufweisen.

6. Anordnung nach einem der Ansprüche 3 bis 5, **dadurch gekennzeichnet, dass** die Mittel (14) zur Aufnahme eines Peripheriegeräts und die Mittel (16) zum Erkennen eines in den Mitteln zur Aufnahme eines Peripheriegeräts aufgenommenen Peripheriegeräts mit den Mitteln (7) zum Senden einer Adresse zur Identifikation des Peripheriegeräts an die Blutbehandlungsvorrichtung und den Mitteln (10) zum Empfangen der Adresse zur Identifikation des Peripheriegeräts von der Blutbehandlungsvorrichtung derart zusammenwirken, dass nach dem Erkennen eines Peripheriegeräts das Peripheriegerät (5) eine Adresse zur Identifikation an die Blutbehandlungsvorrichtung sendet und die Blutbehandlungsvorrichtung (1) die Adresse zur Identifikation des Peripheriegeräts empfängt.

7. Anordnung nach Anspruch 6, **dadurch gekennzeichnet, dass** die Mittel (7, 10) zum Senden und Empfangen Mittel zur drahtlosen Übertragung von Signalen aufweisen.

8. Anordnung nach Anspruch 7, **dadurch gekennzeichnet, dass** die Mittel (7,10) zum Senden und Empfangen einen Funk-Sender und einen Funk-Empfänger aufweisen.

9. Anordnung nach einem der Ansprüche 1 bis 8, **dadurch gekennzeichnet, dass** die Mittel (20) zum Überprüfen der Zuordnung eines Peripheriegeräts zu einem Patienten Mittel zum Überprüfen aufweisen, ob das Peripheriegerät patientenspezifische Signale zur Überwachung des Patienten empfängt, wobei beim Empfangen patientenspezifischer Daten darauf geschlossen wird, dass das Peripheriegerät erfolgreich dem Patienten zugeordnet worden ist.

10. Anordnung nach Anspruch 9, **dadurch gekennzeichnet, dass** die Mittel (20) zum Überprüfen der Zuordnung eines Peripheriegeräts zu einem Patienten mit Mitteln (18) zum Messen der Zeit nach der Entnahme des Peripheriegeräts aus den Mitteln (14) zur Aufnahme des Peripheriegeräts derart zusammenwirken, dass beim Empfangen patientenspezifischer Daten nur dann auf eine erfolgreiche Zuordnung von Peripheriegerät und Patient geschlossen wird, wenn das Peripheriegerät innerhalb eines vorgegebenen Zeitintervalls dem Patienten zugeordnet worden ist.

11. Anordnung nach Anspruch 10, **dadurch gekennzeichnet, dass** die Mittel (20) zum Überprüfen der Zuordnung eines Peripheriegeräts zu einem Patienten mit Mitteln zur Anzeige (13) der verbleibenden Zeit in dem vorgegebenen Zeitintervall zusammenwirken, innerhalb der eine Zuordnung von Peripheriegerät und Patient zu erfolgen hat.

12. Anordnung nach Anspruch 10 oder 11, **dadurch gekennzeichnet, dass** die Mittel (20) zum Überprüfen der Zuordnung eines Peripheriegeräts zu einem Patienten mit Mitteln (12) zum Geben eines optischen und/oder akustischen Signals zusammenwirken, die derart ausgebildet sind, dass ein optisches und/oder akustisches Signal gegeben wird, wenn innerhalb eines vorgegebenen Zeitintervalls nach der Entnahme des Peripheriegeräts aus den Mitteln (14) zur Aufnahme des Peripheriegeräts das Peripheriegerät (5) nicht erfolgreich der Blutbehandlungsvorrichtung (1) zugeordnet worden ist.

13. Anordnung nach einem der Ansprüche 1 bis 12, **dadurch gekennzeichnet, dass** die Mittel (19) zum Bestätigen der Zuordnung von Patient und Blutbehandlungsvorrichtung Mittel zur manuellen Eingabe aufweisen.

## Claims

1. An arrangement comprising at least two blood treatment apparatuses with an extracorporeal blood circuit and at least two peripheral devices, each of which comprising at least one sensor (6) for measuring a patient's physiological data, one blood treatment apparatus (1) of the at least two blood treatment apparatuses and one peripheral device (5) of the at least two peripheral devices being to be allocated to a patient and the peripheral device to be allocated to the patient being to be allocated to the blood treatment apparatus to be allocated to the patient,
wherein the peripheral devices and the treatment units comprise:
means (3, 4) for allocating a blood treatment apparatus to a patient, the means for allocating a blood treatment apparatus to a patient comprising a system of tubes and/or lines for the connection of the patient to the blood treatment apparatus,
means (7, 10, 14, 16) for allocating a peripheral device to a blood treatment apparatus,
means (20) for checking the allocation of a peripheral device to a patient, the means for checking the allocation of the peripheral device to a patient comprising means (7) for sending a signal to the blood treatment apparatus after the successful allocation of the peripheral device to the patient and means (10) for receiving the signal by the blood treatment apparatus,
means (19) for acknowledging that the patient to whom the peripheral device has been allocated is the patient to whom the blood treatment apparatus has been allocated to which the peripheral device has been allocated and
means (17) for releasing the peripheral device only on condition that the correct allocation of the peripheral device and patient has been acknowledged.

2. The arrangement according to claim 1, **characterised in that** the means for allocating a peripheral device to a blood treatment apparatus comprise means (7) for sending an address for identification of the peripheral device to the blood treatment apparatus and means (10) for receiving the address for identification of the peripheral device by the blood treatment apparatus.

3. The arrangement according to claim 1 or 2, **characterised in that** the means for allocating a peripheral device to a blood treatment apparatus comprise means (14) for accommodating a peripheral device and means (16) for detecting a peripheral device accommodated in the means for accommodating a peripheral device.

4. The arrangement according to claim 3, **characterised in that** the means (14) for accommodating a peripheral device are an accommodation unit into which the peripheral device is placed.

5. The arrangement according to claim 3 or 4, **characterised in that** the means (14) for accommodating a peripheral device comprise means (15) for charging a battery (8) for the power supply of the peripheral device.

6. The arrangement according to any one of claims 3 to 5, **characterised in that** the means (14) for accommodating a peripheral device and the means (16) for detecting a peripheral device accommodated in the means for accommodating a peripheral device cooperate with the means (7) for sending an address for identification of the peripheral device to the blood treatment apparatus and the means (10) for receiving the address for identification of the peripheral device by the blood treatment apparatus in such a way that, after detection of a peripheral device, the peripheral device (5) sends an address for identification to the blood treatment apparatus and the blood treatment apparatus (1) receives the address for identification of the peripheral device.

7. The arrangement according to claim 6, **characterised in that** the means (7, 10) for sending and receiving comprise means for the wireless transmission of signals.

8. The arrangement according to claim 7, **characterised in that** the means (7, 10) for sending and receiving comprise a radio transmitter and a radio receiver.

9. The arrangement according to any one of claims 1 to 8, **characterised in that** the means (20) for checking the allocation of a peripheral device to a patient comprise means for checking whether the peripheral device is receiving patient-specific signals for monitoring the patient, whereby it is concluded when patient-specific data are received that the peripheral device has been successfully allocated to the patient.

10. The arrangement according to claim 9, **characterised in that** the means (20) for checking the allocation of a peripheral device to a patient cooperate with means (18) for measuring the time after removal of the peripheral device from the means (14) for accommodating the peripheral device in such a way that, when patient-specific data are received, it is concluded that there is a successful allocation of peripheral device and patient only when the peripheral device has been allocated to the patient within a preset time interval.

11. The arrangement according to claim 10, **characterised in that** the means (20) for checking the allocation of a peripheral device to a patient cooperate with means for displaying (13) the remaining time in the preset time interval within which an allocation of the peripheral device and patient has to take place.

12. The arrangement according to claim 10 or 11, **characterised in that** the means (20) for checking the allocation of a peripheral device to a patient cooperate with means (12) for emitting an optical and/or acoustic signal, said means being designed in such'a way that an optical and/or acoustic signal is emitted if, within a preset time interval after removal of the peripheral device from the means (14) for accommodating the peripheral device, the peripheral device (5) has not been successfully allocated to the blood treatment apparatus (1).

13. The arrangement according to any one of claims 1 to 12, **characterised in that** the means (19) for acknowledging the allocation of patient and blood treatment apparatus comprise means for manual input.

## Revendications

1. Ensemble constitué d'au moins deux dispositifs de traitement du sang comportant une circulation sanguine extracorporelle et au moins deux appareils périphériques qui présentent respectivement au moins un capteur (6) pour la mesure de données physiologiques du patient, dans lequel un dispositif de traitement du sang (1) des au moins deux dispositifs de traitement du sang et un appareil périphérique (5) des au moins deux appareils périphériques sont à affecter à un patient et l'appareil périphérique à affecter au patient est à affecter au dispositif de traitement de sang à affecter au patient,
dans lequel les appareils périphériques et les dispositifs de traitement du sang présentent :
des moyens (3, 4) pour affecter un dispositif de traitement du sang à un patient, qui présentent un système de tubes et/ou de conduites pour relier le patient au dispositif de traitement de sang,
des moyens (7, 10, 14, 16) pour affecter un appareil périphérique à un dispositif de traitement du sang,
des moyens (20) pour vérifier l'affectation d'un appareil périphérique à un patient, qui comprennent les moyens (7) pour envoyer un signal au dispositif de traitement du sang après l'affectation effective de l'appareil périphérique au patient et des moyens (10) pour recevoir le signal du dispositif de traitement du sang,
des moyens (19) pour confirmer que le patient auquel l'appareil périphérique a été affecté, est le patient auquel le dispositif de traitement du sang a été affecté et
des moyens (17) pour valider l'appareil périphérique uniquement à condition que l'affectation appropriée de l'appareil périphérique et du patient ait été confirmée.

2. Ensemble selon la revendication 1, **caractérisé en ce que** les moyens pour affecter un appareil périphérique à un dispositif de traitement du sang présentent des moyens (7) pour envoyer une adresse pour l'identification de l'appareil périphérique au dispositif de traitement du sang et des moyens (10) pour recevoir l'adresse pour l'identification de l'appareil périphérique par le dispositif de traitement du sang.

3. Ensemble selon la revendication 1 ou 2, **caractérisé en ce que** les moyens pour affecter un appareil périphérique à un dispositif de traitement du sang présentent des moyens (14) pour recevoir un appareil périphérique et des moyens (16) pour reconnaître un appareil périphérique reçu dans les moyens pour recevoir un appareil périphérique.

4. Ensemble selon la revendication 3, **caractérisé en ce que** les moyens (14) pour recevoir un appareil périphérique sont une unité de réception dans laquelle l'appareil périphérique est placé.

5. Ensemble selon la revendication 3 ou 4, **caractérisé en ce que** les moyens (14) pour recevoir un appareil périphérique présentent des moyens (15) pour charger une batterie (8) pour alimenter en courant l'appareil périphérique.

6. Ensemble selon l'une quelconque des revendications 3 à 5, **caractérisé en ce que** les moyens (14) pour recevoir un appareil périphérique et les moyens (16) pour reconnaître un appareil périphérique reçu dans les moyens pour recevoir un appareil périphérique coopèrent avec les moyens (7) pour envoyer une adresse pour l'identification de l'appareil périphérique au dispositif de traitement du sang et avec les moyens (10) pour recevoir l'adresse pour l'identification de l'appareil périphérique par le dispositif de traitement du sang de telle sorte qu'après la reconnaissance d'un appareil périphérique, l'appareil périphérique (5) envoie une adresse pour l'identification au dispositif de traitement du sang et le dispositif de traitement du sang (1) reçoit l'adresse pour l'identification de l'appareil périphérique.

7. Ensemble selon la revendication 6, **caractérisé en ce que** les moyens (7, 10) pour l'envoi et la réception présentent des moyens pour la transmission sans fil de signaux.

8. Ensemble selon la revendication 7, **caractérisé en ce que** les moyens (7, 10) pour l'envoi et la réception présentent un émetteur radio et un récepteur radio.

9. Ensemble selon l'une quelconque des revendications 1 à 8, **caractérisé en ce que** les moyens (20) pour recevoir l'affectation d'un appareil périphérique à un patient présentent des moyens pour vérifier si l'appareil périphérique reçoit des signaux spécifiques du patient pour la surveillance du patient, dans lequel lors de la réception de données spécifiques du patient, on en conclut que l'appareil périphérique a été affecté avec succès au patient.

10. Ensemble selon la revendication 9, **caractérisé en ce que** les moyens (20) pour vérifier l'affectation d'un appareil périphérique à un patient coopèrent avec des moyens (18) pour mesurer la durée après le retrait de l'appareil périphérique des moyens (14) pour recevoir l'appareil périphérique de telle sorte que, lors de la réception de données spécifiques du patient, on en conclut à un succès de l'affectation de l'appareil périphérique et du patient que si l'appareil périphérique a été affecté au patient dans un intervalle temporel prédéterminé.

11. Ensemble selon la revendication 10, **caractérisé en ce que** les moyens (20) pour vérifier l'affectation d'un appareil périphérique à un patient coopèrent avec des moyens (13) pour afficher le temps restant dans l'intervalle temporel prédéterminé dans lequel une affectation de l'appareil périphérique et du patient doit être effectuée.

12. Ensemble selon la revendication 10 ou 11, **caractérisé en ce que** les moyens (20) pour vérifier l'affectation d'un appareil périphérique à un patient coopèrent avec des moyens (12) pour émettre un signal optique et/ou acoustique, qui sont conçus de telle sorte qu'un signal optique et/ou acoustique est émis si dans un intervalle temporel prédéterminé après le retrait de l'appareil périphérique des moyens (14) pour recevoir l'appareil périphérique, l'appareil périphérique (5) n'a pas été affecté avec succès au dispositif de traitement du sang (1).

13. Ensemble selon l'une quelconque des revendications 1 à 12, **caractérisé en ce que** les moyens (19) pour confirmer l'affectation du patient et du dispositif de traitement du sang présentent des moyens d'entrée manuels.
